# EUROPEAN PATENT SPECIFICATION

(11) **EP 1 730 264 B1**
(45) Date of publication and mention of the grant of the patent: **04.11.2009**
(21) Application number: 04770637.9
(22) Date of filing: 31.03.2004
(51) Int. Cl.: C12N 5/06, C12N 5/08

(54) **TISSUE-LIKE ORGANIZATION OF CELLS AND MACROSCOPIC TISSUE-LIKE CONSTRUCTS, GENERATED BY MACROMASS CULTURE OF CELLS, AND THE METHOD OF MACROMASS CULTURE**
GEWEBEARTIGE ORGANISATION VON ZELLEN UND MAKROSKOPISCHE GEWEBEARTIGE KONSTRUKTE, DIE DURCH "MAKROMASS-KULTUR" VON ZELLEN HERGESTELLT WERDEN, UND DIE METHODE DER "MAKROMASS-KULTUR"
ORGANISATION DE CELLULES, DE TYPE TISSULAIRE, ET STRUCTURES MACROSCOPIQUES DE TYPE TISSULAIRES, PRODUITES PAR CULTURE MACROMASSIQUE DE CELLULES, ET PROCEDE DE CULTURE MACROMASSIQUE

(43) Date of publication of application: 13.12.2006
(73) Proprietor: Reliance Life Sciences Pvt., Ltd., Mumbai 400 013, Maharashtra (IN)
(72) Inventor: DESHPANDE, Manisha, Sharadchandra;, Sector 17,Vashi Navi Mumbai,Maharashtra (IN); MOJAMDAR, Manoj, Vinoy, Gargaum, Mumbai 400 004 Maharashtra (IN)
(74) Representative: Wright, Simon Mark
(86) International application number: PCT/IN2004/000086
(87) International publication number: WO 2005/095585

(56) References cited:
- US-B2- 6 489 165
- MURAGLIA ANITA ET AL: "Formation of a chondro-osseous rudiment in micromass cultures of human bone-marrow stromal cells." JOURNAL OF CELL SCIENCE, vol. 116, no. 14, 15 July 2003 (2003-07-15), pages 2949-2955, XP002304518 ISSN: 0021-9533
- KIM HYUN WOO ET AL: "An overview of cartilage tissue engineering" YONSEI MEDICAL JOURNAL, vol. 41, no. 6, December 2000 (2000-12), pages 766-773, XP002304519 ISSN: 0513-5796
- JOHNSTONE B ET AL: "IN VITRO CHONDROGENESIS OF BONE MARROW-DERIVED MESENCHYMAL PROGENITOR CELLS" EXPERIMENTAL CELL RESEARCH, SAN DIEGO, CA, US, vol. 238, no. 1, 1998, pages 265-272, XP000881888 ISSN: 0014-4827
- ITOI MANAMI ET AL: "Inductive role of fibroblastic cell lines in development of the mouse thymus anlage in organ culture" CELLULAR IMMUNOLOGY, vol. 183, no. 1, 10 January 1998 (1998-01-10), pages 32-41, XP002304520 ISSN: 0008-8749
- KAPLOWITZ P B ET AL: "STIMULATION OF EMBRYONIC MOUSE LIMB BUD MESENCHYMAL CELL GROWTH BY PEPTIDE GROWTH FACTORS" JOURNAL OF CELLULAR PHYSIOLOGY, vol. 112, no. 3, 1982, pages 353-359, XP009039466 ISSN: 0021-9541
- MACKAY A M ET AL: "CHONDROGENIC DIFFERENTIATION OF CULTURED HUMAN MESENCHYMAL STEM CELL FROM MARROW" TISSUE ENGINEERING, LARCHMONT, NY, US, vol. 4, no. 4, November 1998 (1998-11), pages 415-428, XP002942310 ISSN: 1076-3279
- BUTTERY L D K ET AL: "Differentiation of osteoblasts and in vitro bone formation from murine embryonicstem cells" TISSUE ENGINEERING, LARCHMONT, NY, US, vol. 7, no. 1, February 2001 (2001-02), pages 89-99, XP002978343 ISSN: 1076-3279
- GRAFF RONALD D ET AL: "Role of pericellular matrix in development of a mechanically functional neocartilage." BIOTECHNOLOGY AND BIOENGINEERING, vol. 82, no. 4, 20 May 2003 (2003-05-20), pages 457-464, XP002304521 ISSN: 0006-3592
- COTTRILL C P ET AL: "CELL SORTING AND CHONDROGENIC AGGREGATE FORMATION IN MICROMASS CULTURE" DEVELOPMENTAL BIOLOGY, ACADEMIC PRESS, NEW YORK, NY, US, vol. 122, no. 2, 1987, pages 503-515, XP009038102 ISSN: 0012-1606
- L'HEUREUX N ET AL: "A COMPLETELY BIOLOGICAL TISSUE-ENGINEERED HUMAN BLOOD VESSEL" FASEB JOURNAL, FED. OF AMERICAN SOC. FOR EXPERIMENTAL BIOLOGY, BETHESDA, MD, US, vol. 12, 1998, pages 47-57, XP002942845 ISSN: 0892-6638
- JOON YUNG LEE ET AL: "NEW USE OF A THREE-DIMENSIONAL PELLET CULTURE SYSTEM FOR HUMAN INTERVERTEBRAL DISC CELLS INITIAL CHARACTERIZATION AND POTENTIAL USE FOR TISSUE ENGINEERING" SPINE, PHILADELPHIA, PA, US, vol. 26, no. 21, November 2001 (2001-11), pages 2316-2322, XP001064825

## Description

### 2. FIELD OF THE INVENTION

The present invention relates to tissue engineering. More specifically, this invention relates to generation of three-dimensional tissue-like organization of cells. Further more specifically, this invention relates to the fabrication of three-dimensional macroscopic tissue-like constructs for possible implantation in the human body as a therapy for diseased or damaged conditions.

### 3. BACKGROUND OF THE INVENTION

The human body can be afflicted by several diseased or damaged conditions of different organs, for which one therapeutic approach is the replacement of damaged parts, by extraneously obtained or developed tissue equivalents. For instance, burns or ulcers of the skin can be treated with application of suitable skin equivalents, non-uniting gaps in fractured bone could be treated by implantation.of suitable bone substitutes, and damage to articular cartilage could be repaired by suitable cartilage-forming implants.

Every year, surgeons perform surgical procedures to treat patients who experience organ failure or tissue loss. Surgeons/physicians could treat these patients by transplanting organs from one individual to another, performing reconstructive surgery, or by using mechanical devices such as kidney dialyzers, prosthetic hip joints, or mechanical heart valves. Although these approaches have saved many lives, they are subject to limitations. The limitation of transplantation of organs such as the heart, liver, and kidney is not the surgical technique, but the scarce availability of donor organs.

The possible kinds of naturally available implants have been xenografts obtained from animals, allografts obtained from human donors, and autografts obtained from healthy parts of the patient itself. Xenografts have the problem of immunological non-compatibility and transmission of zoonotic pathogens including retroviruses. Allografts have the problem of immune rejection and non-availability of donors. Autografts have the problem of lack of required amount of suitable tissue and increase in trauma to the patient

For surgical reconstruction, tissue may be moved from one part of the patient to another part These autografts (tissue grafts from the patient) include skin grafts for burns, blood vessel grafts for heart bypass surgeries, and nerve grafts for facial and hand reconstruction. The disadvantages of using autografts also include the need for multiple surgeries and loss of function at the donor site. In addition, surgical reconstruction often involves using the body's tissues for purposes not originally intended and can result in long-term complications.

As a result of these drawbacks of existing therapeutic options, there is a requirement for engineered tissue equivalents, and what has emerged as a new discipline is the science of tissue engineering. Its goals are to create tissues in culture for use not only as model systems in fundamental studies, but more importantly, for use as replacement tissues for damaged or diseased body parts. Although, efforts to generate bioartificial tissues and organs for human therapies go back at least thirty years, such efforts have come closer to clinical success only in the last ten years. This has been made possible by major advances in molecular and cell biology, cell culture technologies, and materials science.

The term "tissue engineering" is relatively recent and has been used more widely in the last five years to describe the interdisciplinary field that applies the principles of engineering and the life sciences toward the development of bioartificial tissues and organs.

One of the major strategies adopted for the creation of lab-grown tissues is the growth of isolated cells on three-dimensional templates or scaffolds (matrices) under conditions that will coax the cells to develop into a functional tissue. When implanted, this bioartificial tissue should become structurally and functionally integrated into the body. The matrices can be fashioned from natural materials such as collagen or from synthetic polymers such as plastics. Ultimately, the scaffold material should be biodegradable over time and should serve as an initial three-dimensional template for tissue growth.

As the cells grow and differentiate on the scaffold, they will produce various proteins needed to recreate a tissue. Degradation of the scaffold ensures that only natural tissue remains in the body. There are also different kinds of bioreactors incorporating different technologies for the task of building a tissue from cells.

Virtually every tissue in the body is a potential target for bioengineering and progress is occurring rapidly on many fronts. For the skin as an organ, different kinds of engineered replacements have been developed - skin has been re-engineered using several different approaches with varying degrees of success.

U.S. Patent No. 5489304 describes a non-cellular graft which has a synthetic outer layer bonded to a collagen-chondroitin sulfate-derived dermal analog layer. This replacement, which is placed initially on the wound before a cultured epithelial autograft is applied, has the disadvantage that it lacks the growth factors important for skin wound healing or the cells that can supply these factors.

U.S. Patent No. 5460939 describes another graft, which is cellular. Here, fibroblasts are grown in bio-resorbable lactic acid / glycolic acid copolymer mesh to form a sheet. In this graft, the scaffolding mesh is not of natural origin.

Eaglstein & Falanga (1997) describe a skin graft, which includes a dermal layer having fibroblasts grown in a bovine collagen matrix. In this graft, extracellular matrix is provided extraneously to the cells, which although manufacture human collagen, but, the extraneous component remains at the time of graft application.

U.S. Patent No.5613982 describes a graft, in which human cadaver skin is processed to remove antigenic cellular components, leaving an immunologically inert dermal layer. This has the limitation of being acellular and of non-availability of human cadaver skin easily.

In all of the above examples, the technological requirements for production of the equivalents are fairly complex, hence would add to the cost of the product. Cellular sheets of fibroblasts using ascorbate have been developed, but the formation of such sheets requires about 35 days (*Michel et al,* 1999; L'Heureux *et al,* 1998).

Thus, there exists a need for the development of a dermal equivalent, the materials for which are easily available, which has no synthetic or natural extraneous matrix that could cause an inflammatory reaction in some patients, which is cellular so that it can produce growth factors and other proteins, which can be prepared in a relatively shorter time, and the preparation of which is technologically simple so that the product is more cost-effective.

An area that requires attention in the field of tissue-engineered products is bone substitutes for patients whose fractures do not heal, leaving non-uniting gaps. Autologous bone grafting increases the trauma to the patient. Different approaches are being tried in bone engineering (Service, 2000). Biomaterials such as collagen matrix infused with growth factors that trigger bone formation have been tried, but such constructs lack the cellular component and the incorporation of the required substantial amount of growth factors makes it a very expensive alternative. Ceramic or hydroxyapatite matrices seeded with mesenchymal stem cells are other approaches, but the use of such scaffolds may not be ideal for the human body. Thus, there exists a need for cost-effective cellular implants which would cause the healing of bone.

Another area that requires attention in the field of tissue-engineered products is cartilage repair. It is a known fact that articular cartilage has limited capacity for complete repair after injury. The cell-based therapy of autologous chondrocyte implantation has shown good clinical results (McPherson & Tubo, 2000) but there remains ample scope for improvement because, the time for complete repair is very long. Possibly, a pre-formed tissue rather than cell suspension would give better results upon implantation. Also, a preformed tissue has an advantage over free cells for surgical implantation. Therefore, various approaches are being tried in making a cartilage-like construct using cells and scaffold, but an ideal scaffolding matrix that will allow the cells in the implant to closely mimic the natural cartilage formation process remains a challenge (Kim & Han, 2000). Thus, there exists a need for developing a preformed tissue that could efficiently initiate cartilage repair when implanted at the site of injury, and which would also be cost-effective.

To summarize, there is a requirement for developing relatively inexpensive living cellular tissue substitutes for therapeutic purposes. The technologically complex bioreactors mentioned earlier for developing three-dimesional tissues are expensive methodologies. Also, in general, there is always a need for the development of tissue substitutes by new methods, which when tested, could prove to have better performance in one or more respects than existing replacements.

Looking to the need of the hour, the scientists of the present invention, have developed novel three- dimensional macroscopic tissue-like constructs which have potential to be used as tissue replacements in human body. A novel characteristic of these tissue-like constructs is that, no scaffold or extraneous matrix is required for tissue generation, the tissues can be formed of completely cellular origin. Also, no other agents that aid in tissue formation (except high cell-seeding-density) such as tissue- inducing chemicals, tissue-inducing growth factors, substratum with special properties, rotational culture are employed for tissue formation. There are no specific complex medium requirements for tissue-like construct formation. The factor causing macroscopic tissue-like construct formation is, large scale culture of cells at high cell seeding per unit area or space.

A crucial aspect of tissue engineering is how to make cells assemble into a tissue or three-dimensional structure. The present invention gives a novel method to achieve the same.

### 4. OBJECTS OF THE INVENTION

In the light of the above, it is therefore an object of the present invention to provide a novel method of assembling cells into three-dimensional tissue-like organization and tissue-like constructs.

The invention therefore provides a method for the generation of a living tissue-like organisation of cells, including three-dimensional tissue-like constructs, without the aid of scaffold or extraneous matrix, the method comprising:
establishing a culture system in which cells that are not embryonic stem cells are seeded at a high density per unit area of a culture vessel 3.33x10⁵ to 3x10⁶ at cells per cm² resulting in a three-dimensional tissue-like formation or organisation of cells, without the aid of any agents that aid in tissue formation, such agents comprising tissue-inducing chemicals, tissue-inducing growth factors, substratum other than unmodified tissue culture plastic, or rotational culture; and
providing said tissue like constructs wherein
optionally the cells are mesodermal cells.

The method provides three-dimensional macroscopic tissue like constructs suitable for possible implantation in the human body as a therapy for diseased or damaged conditions.

The macroscopic tissue-like constructs are histologically competent. By "histological competence" it is meant that these tissue-like constructs can be sectioned easily without disruption.

The method provides three-dimensional tissue-like organization of cells and cost-effective putative tissue equivalents made from fibroblastic cells of mesenchymal origin (at least), such as an engineered putative dermal equivalent made from dermal fibroblasts, putative substitute with bone-like properties made from adipose stromal cells-derived osteogenic cells or from osteoblasts and putative substitute for cartilage repair made from chondrocytes. The method provides other tissues also, which can be constructed from the corresponding cell types, if these other cell types have the properties enabling them to undergo tissue-like mass formation upon macromass culture as described herein.

The method provides three-dimensional tissue-like organization of cells and macroscopic tissue like constructs without using scaffold or extraneous matrix or complex bioreactor for tissue generation.

The method provides three-dimensional tissue-like organization of cells and macroscopic tissue like constructs without using any agents that aid in tissue formation such as tissue-inducing chemicals, tissue-inducing growth factors, substratum with special properties, rotational culture, etc.

The method provides three-dimensional tissue-like organization of cells and macroscopic tissue like constructs of different kinds, formed by using high cell seeding density per unit area or space of culture vessel, without requirement for any other agent that aids in tissue formation.

The method provides macroscopic tissue-like constructs which have a high cell density in the final form.

The method provides tissue-like organization of cells and macroscopic tissue-like constructs which can be formed without the requirement of specific complex medium components.

The properties of the tissue-like organization of cells and macroscopic tissue-like constructs can be modulated to include desired properties by suitable change/s in the growth and/or tissue-forming medium.

The method provides tissue-like organization of cells and macroscopic tissue-like constructs, which can be formed by macromass culture on different compatible growth surfaces according to requirement.

Macroscopic tissue-like constructs can be scaled-up to larger sizes by simple scaling-up in two dimensions of the method used for their formation, viz., macromass culture.

Three-dimensional tissue-like organization may be produced at the microscopic level.

### 5. DESCRIPTION OF THE INVENTION

In the present invention, there is provided a method for the assembly of cells into three-dimensional tissue-like organization by macromass culture, and the novel method of macromass culture. The methods provided produce macroscopic three-dimensional tissue-like constructs that are histologically competent, generated by macromass culture of cells. The present invention relates to tissue engineering. More specifically, this invention relates to fabrication of three-dimensional tissue like constructs suitable for possible implantation in the human body as a therapy for diseased or damaged conditions. This invention gives a method for the organization of cells into three-dimensional tissue-like forms and describes the tissue-like forms themselves.

Fabrication of tissues is a goal important for the replacement of diseased tissues in the human body. Efforts are being made to explore and recruit the tissue-forming abilities of cells for tissue engineering.

The process of tissue engineering of cellular grafts involves the following two (2) major steps -
i. procuring the cells from suitable sources. The procured cells could require suitable preparation such as differentiation into the desired cell type.
ii. constructing the tissue using suitably prepared cells to produce different tissue engineered products.

The present invention addresses the second of these steps. The inventors have developed a simple and cost effective method for the generation of three-dimensional tissue-like organisation of cells and formation of living, cellular, putative tissue substitutes.

The tissue-like constructs produced by the methods of the present invention have the cohesive strength to be able to withstand physical manipulation and handling as would be required for the procedure of placing them surgically at the required site in the body from the container holding them, with the aid of appropriate supporting and handling devices or instruments.

Substantial amount of work has been done till date, in the generation of tissue substitutes that are scaffold-based - these include a scaffold as an important structural and often functional component This scaffold requires to have properties of biocompatibility, biodegradability (so that eventually only natural tissue remains in the body) and of providing a permissive environment for optimal cellular function. The development of scaffolds that are ideal in all possible respects remains a challenge. The present invention has the advantage that it circumvents the need to incorporate a scaffold because the three-dimensional tissue-like constructs generated by the methods of present invention are made without the aid of a scaffold. Formation of histologically competent tissue-like constructs by the macromass method of the present invention does not require a scaffold. Thus the tissue-like constructs produced by the methods of the present invention also eliminate any adverse effects or drawbacks that could be associated with the use of a scaffold which is less than ideal in any respect. In the present invention, extracellular matrix is synthesized by the cells themselves, there are no extraneous matrix components used. Tissue formation takes place simply by seeding the cells at a high cell density per unit area or space of culture vessel. This has been termed as "macromass" culture which is defined as a culture system for three-dimensional tissue-like formation or organization of cells, in which, cells are seeded at a high density per unit area or space of a culture vessel in a range spanning 3·33x10⁵ to 3x10⁶ cells per cm² and there is no requirement for any other agents that aid in tissue formation. A broader definition of macromass culture is a method of generating three-dimensional tissue-like organization, macroscopic or microscopic, from cells by high-density cell seeding, bringing cells together in close proximity in a certain favorable range of high densities of cells in three-dimensional space, that favors cohesive integration of cells into a three-dimensional tissue-like state, there being no requirement for any other agents that aid in tissue formation. A certain high seeding density of cells within a favorable range is required to be achieved within a give space. In the macromass range of favorable high cell seeding densities, when the cells are settled together within the three-dimensional space that is occupied by the cells at the base of the culture vessel, they come into a state of close proximity with one another that triggers or signals them into a tissue formation mode by which they become cohesively integrated. (It may be noted that the macromass range of cell seeding density could be achieved in a vessel with a flat or curved base)The result of using a culture vessel about 0.75 cm in diameter or larger for macromass culture is the formation of macroscopic three-dimensional tissue-like constructs, wherein "macroscopic" means that the size of the tissue is *at least* such that it can be easily visually discerned by the normal unaided human eye.

A previously known tissue culture system, high-density *micromass* culture has been used for the chondrogenic differentiation of cells, and the scale of such culture has been limited to being 10 to 20 µl spots of cell suspension (Yoon *et al,* 2000). Classically, limb mesenchymal cells when cultured *in vitro* as micromass cultures, undergo formation of precartilage condensations or aggregates which are present as individual nodules covering the area of the micromass spot (Ahrens et al, 1977). The cell nodules thus formed are separate from one another with cells not formed into nodules in between and are microscopic. The larger, yet microscopic, spheroidal structures in which all the cells come together to form one aggregate are generated with the requirement of specific components added to the culture medium such as growth factors, as mentioned later in the text. However, the tissue-like masses generated by the present inventors are *macroscopic (and formed without the aid of any specific agent that aids* in *tissue formation),* and thus possess the desirable quality of size required to have potential as tissue replacements for the human body. In the tissue-like organization by macromass culture of the present invention, all cells become part of the integrated tissue-like organization which is thus whole; there are no individual nodules. It has been earlier found that, by micromass culture, leg precartilage mesenchymal cells produced a nodular pattern (Downie & Newman, 1994). While wing precartilage mesenchymal cells produced a sheet pattern by micromass culture; this was in a serum-free culture system, unlike the macromass culture system of the present invention. Also, the leg precartilage mesenchymal cells could produce a sheet-like pattern, but this was upon treatment with TGFβ1 in serum-free medium, again unlike macromass culture, wherein no specific agent that aids in tissue formation is required for tissue-like sheet formation and there is no requirement for serum-free conditions.

Hitherto, the question whether cell-cell aggregation leading to whole tissue-like mass formation will occur by high cell-seeding-density culture without any specific agents that aid in tissue formation in the medium had not been investigated. However, the work of the present inventors has addressed this question and the present invention answers in the affirmative.

High-density culture has been used to induce chondrogenesis with microscopic individual nodule formation, but has not been assessed so far, on the larger macroscopic scale for generation of macroscopic tissues for replacement in the human body. And even on the microscopic scale, as mentioned above, whole aggregates are formed only with the help of specific agents, unlike macromass culture of the present invention.

Although the term "macromass" at first perception may appear to mean a mere extension of "micromass", it is actually different in the important respect that micromass has been developed as a method for chondrogenic differentiation of cells and also includes specific complex medium requirements for even microscopic whole spheroidal aggregate formation (as mentioned below), while macromass is a method for the generation of three-dimensional tissue-like organization of cells and macroscopic tissue-like constructs, and without specific complex medium requirements for formation.

Till date, efforts have been made towards development of cellular aggregates, the results of which have been microscopic masses termed spheroids. Spheroids are three-dimensional cellular structures that have been made from hepatocytes and other cells with the help of a variety of agents that aid in tissue-like formation like non-adherent dishes (Takezawa *et al,* 1993), spinner-flask culture (Abu-Absi *et al,* 2002), polymeric substances like Eudragit (Yamada *et al,* 1998), Matrigel (Lang *et al,* 2001), Primaria dishes (Hamamoto *et al,* 1998), poly-D-Lysine coated dishes (Hamamoto *et al,* 1998), proteoglycan coating (Shinji *et al,* 1988), culture medium flow (Pollok *et al,* 1998), rotational culture (Furukawa *et al,* 2001), liquid overlay technique (Davies *et al,* 2002), factors enhancing cell-cell adhesion such as insulin, dexamethasone & fibroblast growth factor (Furukawa *et al,* 2001), aggregation-promoting polymer-peptide conjugates (Baldwin & Saltzman, 2001), rotating-wall bioreactor (Baldwin & Saltzman, 2001), etc. Unlike these spheroids, the tissue masses made in our work are generated without the aid of any such agent that aids in tissue-like formation. The above mentioned spheroids are much smaller, being mostly in the micrometer or sub-millimeter range. Since it is possible to make macroscopic tissue masses by macromass culture as described in the present invention, these have a clear advantage over spheroids for placement in required locations in the human body.

The largest of spheroids ( about 1 mm in diameter ) the present inventors have found in published literature was formed by high-density pellet culture (Mackay *et al,* 1998). Their formation took place in the presence of a serum-free defined medium containing TGF-β3, dexamethasone, ascorbate 2-phosphate and insulin-transferrin-selenium supplement where as such a serum-free defined medium is not required for tissue generation by macromass culture. In the preceding report using pellet culture of bone-marrow derived mesenchymal progenitor cells, it had been found that spheroidal aggregate formation did not take place in the pellets incubated in DMEM + 10% FCS (Johnstone *et al,* 1998), while tissue-like constructs by macromass culture form in DMEM + 10% FCS. Spheroid formation by micromass culture of multipotential mesenchymal cells has been reported; here again spheroidal aggregate formation took place only upon treatment of the micromass culture with TGFβ1 or bovine bone extract (Denker *et al,* 1995). Microscopic bone cell spheroids have been reported to form in the presence of serum-free medium containing TGFβ1 (Kale *et al,* 2000; US Patent Application no. 20020127711) and culture of cells in the absence of serum and in the presence of TGFβ1 is essential for bone cell spheroid formation in this method as reported. In the above work, cell culture densities of about 1x10³ cells/cm² to 1x10⁶ cells/cm² have been described as being favorable for spheroid formation. In addition to the other features of the tissue-like constructs of the present invention that distinguish it from the above work, namely, being macroscopic, not requiring absence of serum & not requiring TGFβ1 for formation, the favorable seeding density range also is different. In our work, tissue-like construct does not occur at 1x10⁵ cells/cm² or below, while 1x10³ cells/cm² of the above work is a 100-fold lower. Cell-aggregates or nodules of osteogenic embryonic stem cells have been formed (Buttery *et al,* 2001), but again, these nodules were microscopic and not formed by high cell-seeding-density-culture as described in the present invention. The tissue masses produced by the methods of the present invention, that can be generated by macromass culture when done on a large scale, are macroscopic, hence magnitudes much larger than any spheroids that have been developed, and have no such specific complex medium requirements for formation. A simple medium such as DMEM + 10% FCS suffices for tissue-like construct formation by macromass culture. Cohesive plugs of cells have been earlier formed from chondrogenic cells (US Patent No. 5,723,331), but a preshaped well having a surface that discourages cell attachment and thus deprives cells of anchorage is a critical requirement for this, whereas, in the present invention, there is no requirement for such a surface for tissue-like construct formation.

In one embodiment of the methods of the invention, a tissue-like sheet is formed from human dermal fibroblasts as a potential dermal substitute. The dermal fibroblasts can be of allogeneic origin, since it is known that human dermal fibroblasts are relatively non-immunogenic upon transfer to an allogeneic recipient (U.S. Patent no. 5460939). This tissue-like sheet has the potential to be a dermal equivalent, the materials for which are easily available, which has no synthetic or natural extraneous matrix that could cause an inflammatory reaction in some patients, which is cellular so that it can produce growth factors and other proteins, which can be prepared in a relatively shorter time, and the preparation of which is technologically simple so that the product is more cost-effective.

In another embodiment of the methods of this invention, by macromass culture, a tissue-like mass with bone-like properties is generated from adipose stromal cells-derived osteogenic cells, as a putative tissue substitute that could have the potential to cause healing of small non-uniting gaps in bone fractures. This could be a possible autologous therapy. This tissue-like mass could have the potential to be a cost-effective cellular implant, devoid of extraneous scaffold, which could cause the healing of small gaps in bone. In this embodiment of the methods of this invention, a tissue-like construct has also been made from bone-derived osteoblasts.

In yet another embodiment of the methods of this invention, a tissue-like sheet has been developed from human chondrocytes, as a putative implant inducing cartilage repair in patients with articular cartilage damage. Autologous chondrocytes could be used for this tissue-therapy, since chondrocytes can be obtained from small biopsies of cartilage. This tissue-like sheet could have the potential to be a preformed tissue that could efficiently initiate cartilage repair when implanted at the site of injury, and which would also be cost-effective.

In an additional embodiment of the methods of this invention, microscopic three-dimensional tissue-like organization is generated by macromass culture within a gelatin sponge.

To summarize, tissue-like constructs produced by the methods of the present invention, generated by macromass culture could have the potential to be living, cellular, tissue substitutes that are free of scaffolds and of extraneous extracellular matrix, and that are technologically simple to make and hence would be cost-effective. The tissue-like constructs produced by the methods of the present invention, described in detail as tissue substitutes for therapeutic purposes, could also find other applications as well, such as in *vitro* drug testing and the like.

### 6. BRIEF DESCRIPTION OF THE DRAWINGS:

Preferred embodiments of the methods of the present invention are further illustrated in the accompanying figures, as described below -
- **FIG 1.**: Photographs showing the macroscopic tissue-like constructs formed by macromass culture, in 3.5 cm dishes, from (a) dermal fibroblasts (b) adipose stromal cells (c) chondrocytes (d) osteoblasts.
- **FIG 2.**: Cell-cell adhesion process resulting in tissue-like construct formation taking place in macromass culture of adipose stromal cells (a) One hour after start of macromass culture (b) Six hours after start of macromass culture.
- **FIG 3.**: Histological examination of tissue-sheet formed by macromass culture of dermal fibroblasts (a) Hematoxylin & eosin staining shows the three-dimensional organization (b) Masson-Trichome staining shows collagen synthesis.
- **FIG 4.**: Histological examination of tissue-like construct formed by macromass culture of osteogenic cells derived from adipose stromal cells (a) Hematoxylin & eosin staining shows three-dimensional organization (b) Masson-Trichome staining.
- **FIG 5.**: Histological examination of tissue-like construct formed by macromass culture of chondrocytes (a) Hematoxylin & eosin staining shows three-dimensional organization (b) Masson-Trichome staining.
- **FIG 6.**: Collagen type I immunostaining of histological section of tissue-like construct made from dermal fibroblasts, showing positive detection of collagen type I.
- **FIG 7.**: Cells regrown from dissociated tissue sheet made from dermal fibroblasts by macromass culture for assessing viability.
- **FIG 8.**: Gene expression analysis of tissue sheet formed from dermal fibroblasts by macromass culture, assayed by Reverse Transcriptase-PCR. The RT-PCR products corresponding to various genes known to be important for the wound healing process of the skin are shown electrophoresed on 2 % agarose gel (M) DNA molecular size marker (1) Collagen type I (2) Syndecan 2 (3) Tenascin-C (4) Vascular endothelial growth factor (5) Collagen type III (6) Fibronectin (7) Keratinocyte growth factor (8) Transforming growth factor 1β.
- **FIG 9.**: Gene expression in tissue-like construct made from osteogenic cells derived from adipose stromal cells. The RT-PCR products are shown electrophoresed on 2% agarose gel (M) DNA molecular size marker (1) Collagen type I (2) Osteopontin (3) Parathyroid hormone receptor (4) Bone morphogenetic protein 2 (5) Bone morphogenetic protein 4 (6) Bone morphogenetic protein receptor IA (7) Bone morphogenetic protein receptor IB.
- **FIG**: **10.** Gene expression in tissue-like construct made from chondrocytes. The RT-PCR products are shown electrophoresed on 2% agarose gel (M) DNA molecular size marker (1) Aggrecan (2) Collagen type II (3) Collagen type X.
- **FIG 11.**: Histological analyses of tissue-like construct made from osteogenic cells derived from adipose stromal cells in the presence of conditioned osteogenic medium showing (a) focal actual bone formation within the tissue-like construct (Masson-Trichome) and (b) focal calcium deposition (Von Kossa), demonstrating that the properties of the tissue-like constructs made by macromass culture can be modulated by changes in the medium.
- **FIG 12.**: Histological sections (Toluidine Blue staining) of tissue-like constructs made from chondrocytes in the presence of (a) DMEM + 10% FCS (b) Chondrogenic medium, showing cartilage-specific extracellular matrix formation in (b), demonstrating that the properties of the tissue-like constructs made by macromass culture can be modulated by changes in the medium.
- **FIG 13.**: Histological section (Masson-Trichome staining) of composite object consisting of tissue-like sheet made from dermal fibroblasts and a collagen+fibrin gel, demonstrating that tissue-like organization made by macromass culture can be a component of an object.
- **FIG 14.**: Histological section (Hematoxylin & eosin staining) of macromass culture within a gelatin sponge, showing clusters of microscopic three-dimensional tissue-like organizations formed.

Various other aspects of the invention are described in further details in the following sections.

### 7. MATERIALS AND METHODS :

It is clarified by the inventors of the present invention that, throughout the entire description of this invention and in the appended claims, although area of culture vessel has been referred to in terms of diameter of a circular culture vessel, the aspect being described actually includes a culture vessel of any shape, its area being same as that of a circular vessel of the mentioned diameter. Also, macromass culture could be achieved in a culture vessel with a flat or non-flat base, although work presented in this description relates to a vessel with a flat base.

### 7.1. Cell isolation, media and culture -

In the methods of present invention, human dermal fibroblasts were isolated from human skin biopsies. The dermis was separated from the epidermis by treatment with Dispase (Sigma, St. Louis, USA). The dermis was minced and digested with 0.01 % collagenase in DMEM + 10 % FCS overnight and then cells were allowed to attach. Cells were cultured in DMEM + 10 % FCS at 37°C in 5% CO₂ and subcultured using Trypsin-EDTA solution. Adipose stromal cells were isolated from human liposuction material according to the protocol described by Zuk *et al* (2001). These cells were maintained in DMEM + 10 % FCS. These cells were induced into osteogenic differentiation according to the protocol described by Zuk *et al* (2001). Chondrocytes were isolated from human cartilage fragment by mincing the cartilage and treating with collagenase before incubating in the maintenance medium of DMEM + 10 % FCS. Osteoblasts were isolated from human bone by a similar procedure and maintained in DMEM + 10% FCS having 50 µg/ml ascorbic acid. Conditioned osteogenic medium was prepared by using the medium in which osteogenic adipose stromal cells were being grown as part of the osteogenic medium for the same cells in the next subculture. Chondrogenic medium was prepared according to Zuk et al (2001).

### 7.2. Tissue-like construct formation -

Formation of tissue-like constructs was achieved by macromass culture, which is the novel method of the present invention, earlier defined in the present description of this invention. Cultured cells were harvested using Trypsin-EDTA. They were resuspended in appropriate volume of medium and seeded preferably in culture dishes with a well diameter of 3.5 cm at a seeding density of about 10⁶ cells per cm² or in the macromass favorable range of tissue-forming cell densities. Thus, preferably, about 10⁷ cells total were seeded in a single well of a six well plate (9.6 cm² area) for the formation of a single tissue. For smaller or larger tissue-like constructs, the total number of cells seeded was adjusted so as to achieve seeding density favorable for tissue-like organization.

### 7.3. Histological analyses -

Upon formation, tissue-like constructs were fixed and processed for histological examination. Von Kossa staining and Alcian blue staining were done on the appropriate tissue constructs of the present invention, according to the methods described by Zuk *et al* (2001) and Bancroft *et al* (1994). Oil Red O staining was done by the method described by Bancroft *et al* (1994). Toluidine Blue staining was done as follows - After deparaffinization and hydration of sections, they were stained for 1 minute in 2% aqueous-Toluidine Blue solution, then washed in water for 2-3 mins. The sections were then dehydrated in 2 changes of 100% acetone, then cleared in xylene and mounted.

Other histological procedures were performed by the Histopathology Laboratory at Sir Hurkisondas Nurrotamdas Hospital & Research Centre, Mumbai, India.

### 7.4. Immunohistochemistry -

Collagen type I immunostaining was performed on sections of paraffin-embedded tissue using goat anti collagen type I antibody and the ABC Staining System of Santa Cruz Biotechnology, Santa Cruz, USA.

### 7.5. Viability of cells in tissue-like constructs formed -

The tissue masses produced by the methods of the present invention were minced, digested with 0.5 mg/ml collagenase in serum free DMEM for 15 mins, and the released cells were resuspended in growth medium. An aliquot was stained with Trypan Blue. The cells were seeded in a culture flask to assess viability.

### 7.6. Gene expression analysis -

Gene expression in the tissue-like constructs made from dermal fibroblasts, osteogenic adipose stromal cells and chondrocytes was analyzed by Reverse Transcriptase-PCR. RNA was extracted from the tissue-like construct using Trizol (Gibco-BRL, Grand Island, USA). RT-PCR was performed using primers specific for the respective genes and the Titan One-Tube RT-PCR system (Roche, Mannheim, Germany).

7.7 Collagen + fibrin gel preparation - For preparation of collagen + fibrin gel, 134 µl of 3.33 mg/ml rat tail collagen type I (Sigma, St. Louis, USA) in 0.1 N acetic acid, 8 µl of 4N NaOH, 165 µl of 28.8 mg/ml fibrinogen in 1X DMEM, and 210 µl of 1.48 mg/ml thrombin in 1.66X DMEM were mixed. The mix was allowed to gel for 2 hours at 37°C.

### 7.8 Tissue formation by macromass culture within gelatin sponge -

Gelatin sponge used was AbGel (Sri Gopal Krishna Labs. Pvt. Ltd, Mumbai, India).

### 8. RESULTS AND DISCUSSION:

### 8.1. Formation of three-dimensional tissue-like organization and macroscopic tissue-like constructs -

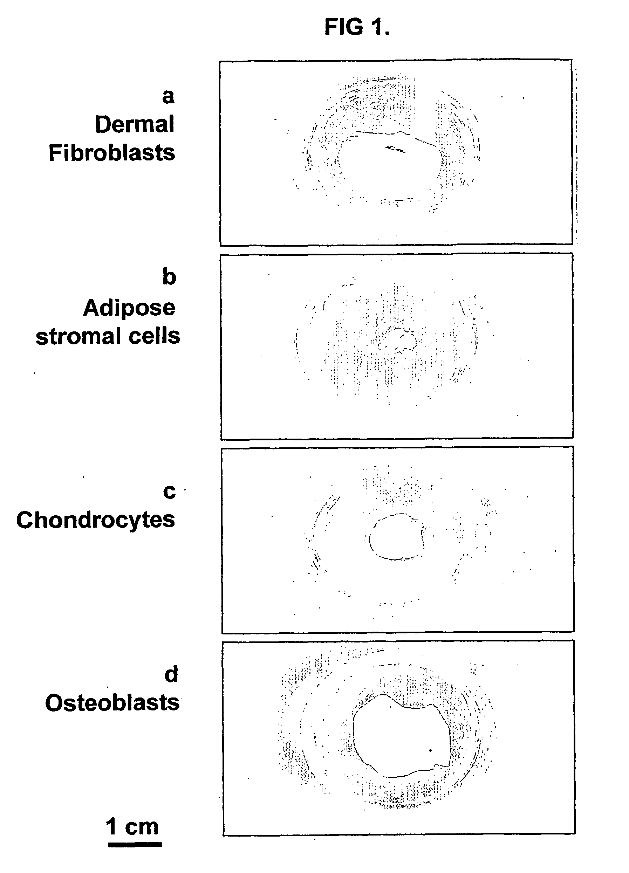

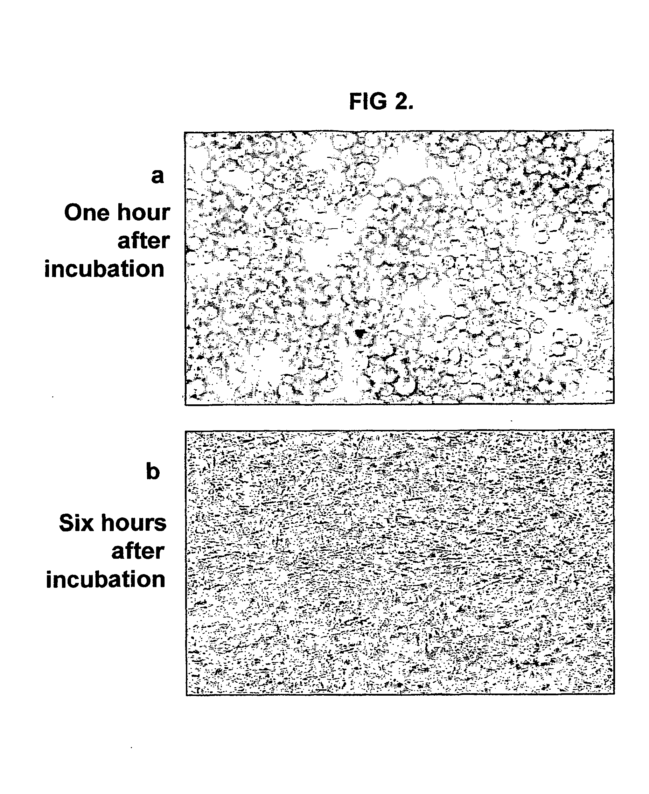

By macromass culture, tissue-like masses (FIG 1) were formed from dermal fibroblasts in the presence of DMEM + 10% FCS in the shape of a sheet which either detached from the growth surface spontaneously or by gentle peeling with a blunt instrument. The adipose stromal cells also formed a tissue-like mass in DMEM +10% FCS which was negative for lipids by Oil Red O staining. Since this was the case, to make possibly useful tissue from adipose stromal cells, they were differentiated into osteogenic cells, which upon macromass culture formed a similar sheet which can contract to a tight mass upon further incubation after detaching. Chondrocytes isolated from human cartilage also formed such a tissue sheet upon macromass culture in DMEM + 10%FCS. Osteoblasts isolated from human bone also formed a tissue-like construct by macromass culture in DMEM + 10% FCS, after washing away the maintenance medium containing ascorbic acid. Integration of cells appears to be playing an important role in such tissue-like construct formation as seen from the extensive formation of extensions from the cells and cell integration, shown in FIG 2. When dermal fibroblast macromass culture was scaled up by seeding cells at the mentioned seeding density in a 8.5 cm petri dish, a much larger sheet formed. Thus, it appears that macromass culture can be directly scaled up areawise to obtain as large tissues as desired. Dermal fibroblasts also formed a sheet by macromass culture in serum free DMEM, but the time of formation was greater than in DMEM containing 10% FCS, overnight compared to 3-4 hours in serum-containing medium. In serum-containing medium, the time of formation of tissue from dermal fibroblasts, from adipose stromal cells & osteogenic cells derived from them was about 4 hour, and from chondrocytes was about 18 hours. Osteogenic cells derived from adipose stromal cells formed a tissue-like mass in osteogenic medium as well as in DMEM + 10% FCS, after washing the cells to remove osteogenic medium containing ascorbic acid. Generation of tissue-like constructs by macromass culture has been done successfully in culture vessels of diameter 0.75 cm to 8.5 cm. It can be extrapolated that macromass culture in culture vessels smaller than 0.75 cm diameter and larger than 8.5 cm diameter would result in formation of smaller or larger tissue-like constructs respectively. Thus, the dimensions of the three-dimensional tissue-like constructs can be varied.

The tissue-like organisation of cells and the different forms thereof, are three-dimensional and have flexibility with respect to dimensions, comprising: different three dimensional sizes; larger or smaller tissue-like constructs achieved by scaling up or down the culture system for three-dimensional tissue-like formation; and variable size or scale by changing the number of cells used to achieve a seeding density within a favorable range of tissue-forming densities.

Although these tissue-like constructs are not fully formed tissues, they could be capable of inducing and participating in the healing process in the body, in a way analogous to the findings that implantation of even stem cells or partially differentiated cells (which are not fully formed tissue but are at the very beginning of tissue formation) can lead to repair and regeneration (Kaji & Leiden, 2001).

It was found that the phenomenon of three-dimensional tissue-like mass formation by macromass culture was dependent on cell seeding density. To examine whether tissue formation took place at all high densities or not, dermal fibroblasts were seeded at a range of different cell densities per unit area. It was found that tangible sheet formation took place at about 3.33 x 10⁵ cell per cm², while at a seeding density of 6.66 x 10⁴ cells per cm² (five times lesser) or lower, no tissue sheet formed. Also, tissue sheet formation occurred at the seeding density of 3 x 10⁶ cells per cm² but not at 7 x 10⁶ cell per cm² or higher, at the latter seeding density the cells only loosely clumped together but did not form a cohesive tissue mass. Thus, tissue sheet formation took place at 3.33 x 10⁵ cell per cm² and at 3 x 10⁶ cells per cm² as well as all densities lying between these two figures that were tested. At the densities tested above or below this range, tissue formation did not occur. Similar experiments were done with native adipose stromal cells and with chondrocytes and the results are tabulated in Table 1. As with dermal fibroblasts, there was a minimum and maximum tissue-like construct forming seeding density for these cell types also, similar to those of dermal fibroblasts. These data indicate that a minimum and maximum cell seeding density per unit area exist for tissue formation by macromass culture. The range of high cell densities at which tissue formation occurs by macromass culture could be different for other cell types not tested. The lower seeding densities of cells used as shown in Table 1 resulted in thinner tissue-like construct, that is, having smaller three dimensions, while the higher seeding densities used resulted in thicker tissue-like constructs, that is, having larger three-dimensions. Thus, the dimensions of the three-dimensional tissue-like constructs can be varied.

**Table 1. Dependence of three-dimensional tissue-like construct formation on cell-seeding density.**

| Total cells plated per cm² | Dermal fibroblasts | Adipose stromal cells | Chondrocytes |
|---|---|---|---|
| 1.3 x 10⁴ | - | - | - |
| 6.0 x 10⁴ | - | - | - |
| 1x10⁵ | - | - | - |
| 3.0 x 10⁵ | + | + | +/-(very weak) |
| 7 x 10⁵ | + | + | + |
| 1x10⁶ | + | + | + |
| 3x10⁶ | + | +/-(less cohesive) | + |
| 7x10⁶ | - | - | - |
| 10 x 10⁶ | - | - | - |

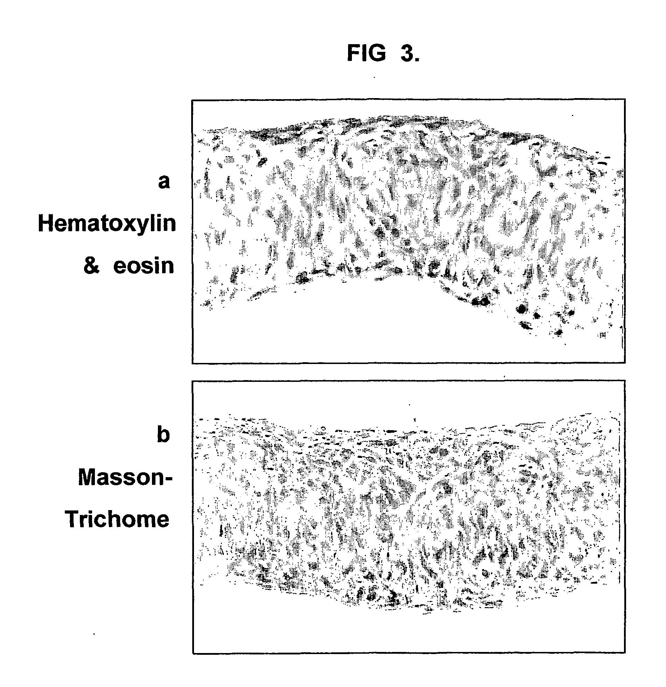

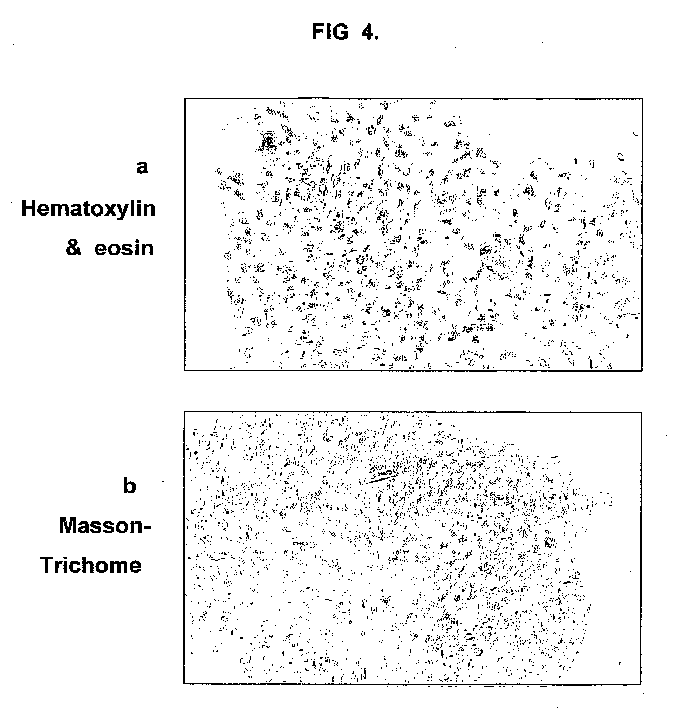

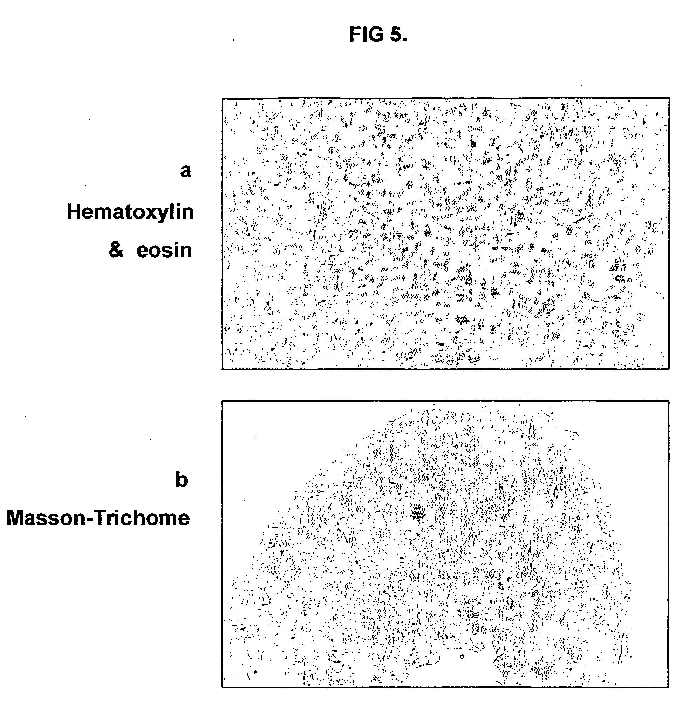

### 82. Histology (FIGS 3,4,5) -

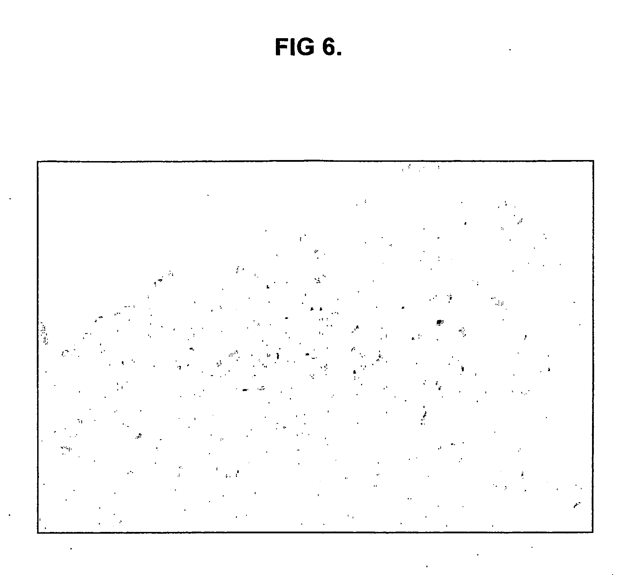

Hematoxylin& eosin staining of the sections of the different tissue-like constructs produced by the methods of the present invention shows three-dimensional structural organization and extracellular matrix formation. Masson-Trichome staining of tissue-like constructs formed from dermal fibroblasts as well as native stromal cells and osteogenic stromal cells showed collagen synthesis. Collagen type I immunostaining of a tissue-like construct made from dermal fibroblast, incubated for 10 days, showed positive staining for collagen type I (FIG 6). Thus, extracellular matrix formation appears to be taking place in tissue-like construct formation by the method of macromass culture.

### 8.3. Viability of cells in tissue-like constructs formed -

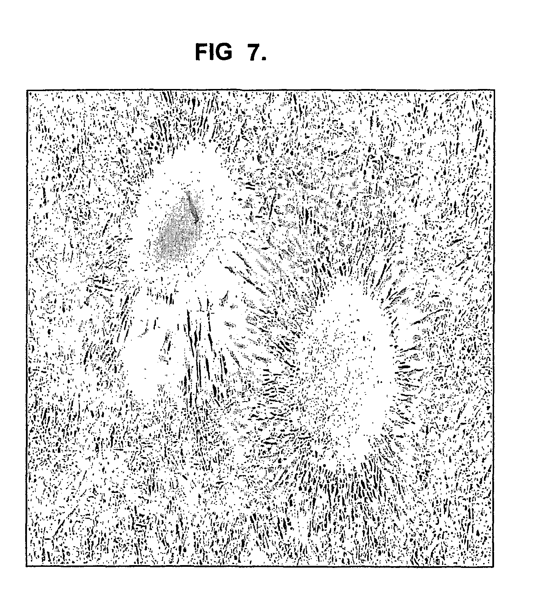

Cells re-isolated from the tissues formed from dermal fibroblasts and chondrocytes had viability greater than 98% by Trypan Blue staining; the cells from tissue mass from adipose stromal cells were about 90% viable. The isolated cells and clumps were plated to assess regrowth, and were found to be viable in each case, as depicted in FIG 7 showing cells growing out of clumps of dissociated dermal fibroblast tissue sheet.

### 8.4. Expression analysis of tissue-like constructs -

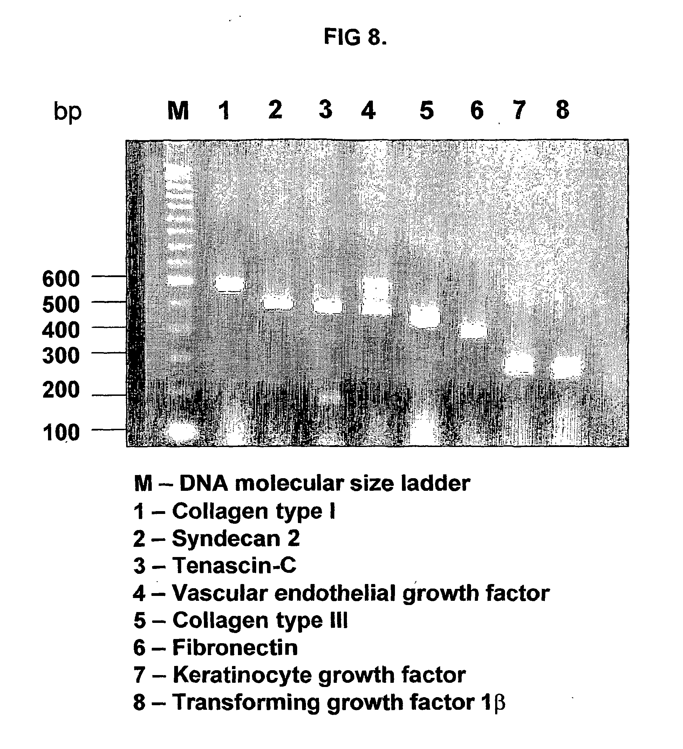

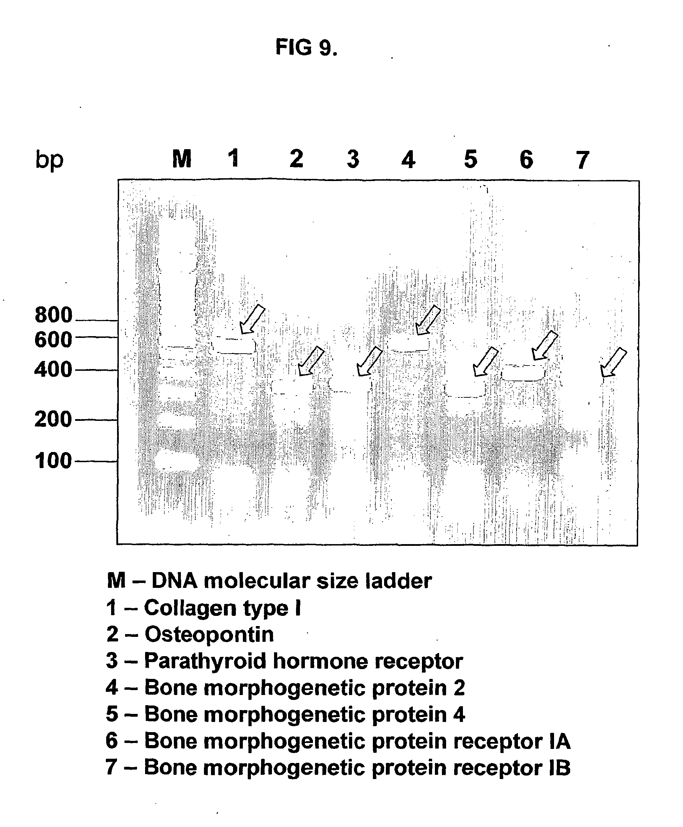

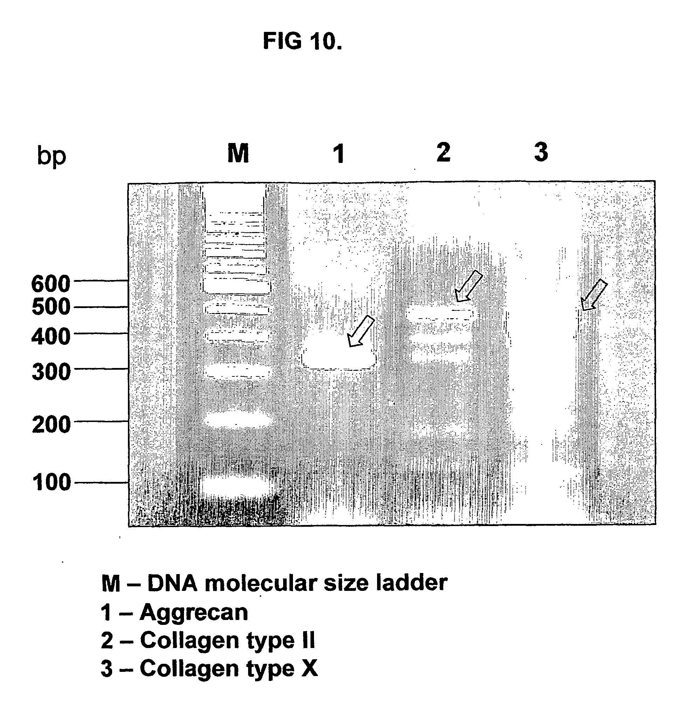

To assess whether the tissue sheet formed from dermal fibroblasts has potential as a dermis substitute, the expression of genes known to play an important role in the wound healing process of the skin was analyzed (FIG 8). Collagen type I, collagen type III, keratinocyte growth factor, TGFβ1, fibronectin, vascular endothelial growth factor, tenascin-C and syndecan-2 were found to be expressed in the tissue sheet, thus demonstrating that this tissue-like sheet made from dermal fibroblasts has potential as a dermis substitute. To assess whether the tissue-like construct made from osteogenic adipose stromal cells had potential has a bone-like tissue substitute, the expression of bone-specific expressed genes was analyzed. The tissue-like construct was found to express collagen type I, osteopontin, parathyroid hormone receptor, bone morphogenetic protein 2, bone morphogenetic protein 4, bone morphogenetic protein receptors IA, IB & II, thus demonstrating its bone-like properties (FIG 9), in addition to the focal calcification and actual bone spicule formation mentioned below. Similarly, the tissue-like construct made from chondrocytes was assessed for its potential as a cartilage tissue substitute. The cardage-specific expressed genes collagen type II, aggrecan and collagen type X were found to be expressed, thus demonstrating its cartilage-like properties (FIG 10), in addition to the formation of cartilage-specific extracellular matrix as mentioned below.

### 8.5. Modulation of properties of tissue-like constructs by flexibility of tissue-formation medium

In macromass culture, it is possible to tailor the properties of the tissue formed by including appropriate medium components or by changing the medium, since, as far as has been tested, tissue formation is independent of medium conditions, as long as something that inhibits tissue-like organization by macromass is not included in the medium. This is apparent from tissue sheet formation by dermal fibroblasts in both serum-containing and serum free medium, as well as from tissue formation from adipose stromal cells in both DMEM + FCS and osteogenic medium, which contains dexamethasone and β-glycerophosphate. Thus, the properties of the tissue formed can be modulated to incorporate desirable properties by modifying the tissue-formation medium and/or growth medium of the cells.

The tissue-like organisation of cells is formed in the presence of different culture media, different growth and/or tissue-formation media; and the tissue-like organisation of cells' properties are modulated by (a) including components in the growth and/or tissue-formation medium, provided that addition of these components does not adversely affect tissue formation, or (b) the medium of the cells is changed, provided that this change in medium does not adversely affect tissue formation.

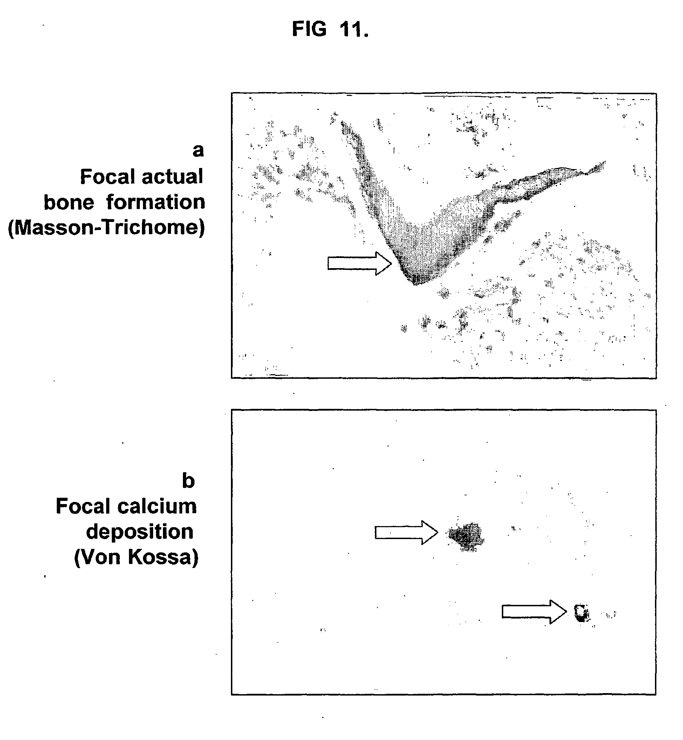

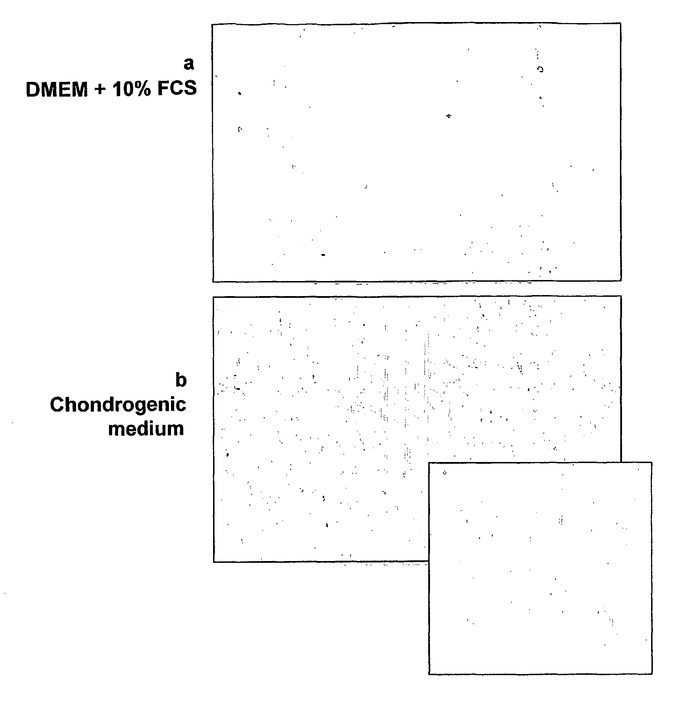

For instance, as presented in this invention, bone-like properties in the shape of actual bone formation were induced in tissue formed from adipose stromal cells by culturing the cells, and forming the tissue-like construct in conditioned osteogenic medium as compared to osteogenic medium alone, where no actual bone spicule formation was seen (FIG 11). Von Kossa staining of the tissue mass formed from osteogenic stromal cells in the presence of conditioned osteogenic medium showed focal regions of calcification, thus demonstrating bone-like properties, while the construct made from osteogenic stromal cells in non-conditioned osteogenic medium did not display these properties. Another example of such modulation is the formation of tissue-like constructs from chondrocytes in the presence of DMEM + 10% FCS or in the presence of chondrogenic medium which contained 1% FCS and insulin; and TGFβ1 as a chondroinductive agent. The property of having extracellular matrix characteristic of the cartilage phenotype was generated in the tissue-like construct made in chondrogenic medium while the one made in DMEM + 10% FCS did not have such a property. This is shown by the Toluidine blue staining in FIG 12. Thus, even as it remains that tissue formation by macromass culture does not have specific complex medium requirements, such specific components can be used in the medium for modulating the properties, of the tissue formed. It also follows from the above results that tissue-like organization and macroscopic tissue-like constructs can be formed in the presence of different culture media, with the different media presented here are examples and do not limit the invention hereof to these examples.

### 8.6. Growth surface for macromass culture -

The tissue-like sheets formed from cells plated on plastic surface had a tendency to detach spontaneously and curl or roll up, which is not desirable, since the sheet does not straighten once rolled up. A tissue being developed as a possible dermis substitute requires to remain straight. For this, macromass culture of dermal fibroblasts was done on a Hybond-N (Amersham Pharmacia Biotech, Buckinghamshire, UK) filter placed in a plastic dish. With this adaptation, the sheet that formed remained straightened and adhered to the filter, so, in the future, it could be applied onto a skin wound with the filter side up. This experiment demonstrates that it can be possible to achieve tissue formation by macromass culture on different compatible growth surfaces. Thus, in this case, the sheet made by macromass culture becomes a component of a putative implant that includes a nylon filter which serves as a supporting layer and its requirement is not as a scaffold for tissue-like organization of the cells. This supporting layer is not designed to integrate into the body along with the dermis-like sheet, and so it is not a scaffold which would become a part of the body, but a supporting handling device for the application of the dermis-like sheet. This supporting layer would be removed after healing.

### 8.7. Tissue-like construct as a component of an object -

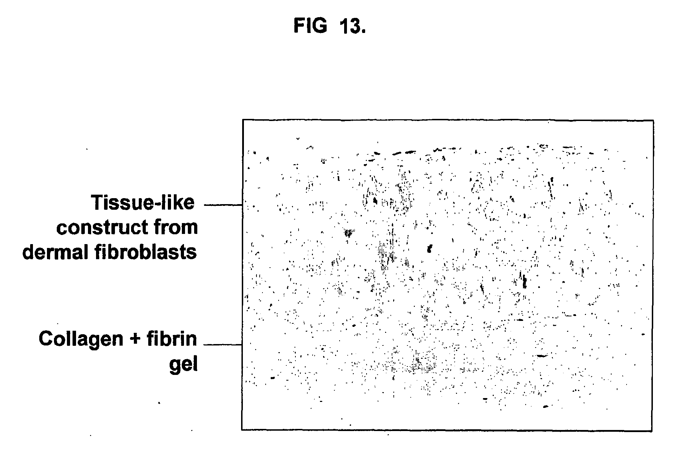

To demonstrate that a tissue-like construct can be incorporated to become a component or part of a (larger) object, dermal fibroblasts were seeded by macromass culture to form a tissue-like sheet, and then after removing the culture medium, it was overlaid with a collagen and fibrin gel mix. The gel was then allowed to set. Now, the gel could be lifted with the tissue-like sheet adhered to one side of it. A histological section of this composite, in which the tissue-like construct is a component, is shown in FIG 13. Apart from gels and sheets, other matrices like sponges or membranes could also support tissue-like-constructs from one side by adhering to them. Thus, tissue-like organization and macroscopic tissue-like constructs can be combined with different objects, for example, a sheet or membrane, a gel, a sponge, or other matrices.

### 8.8. Microscopic three-dimensional tissue-like organization by macromass culture -

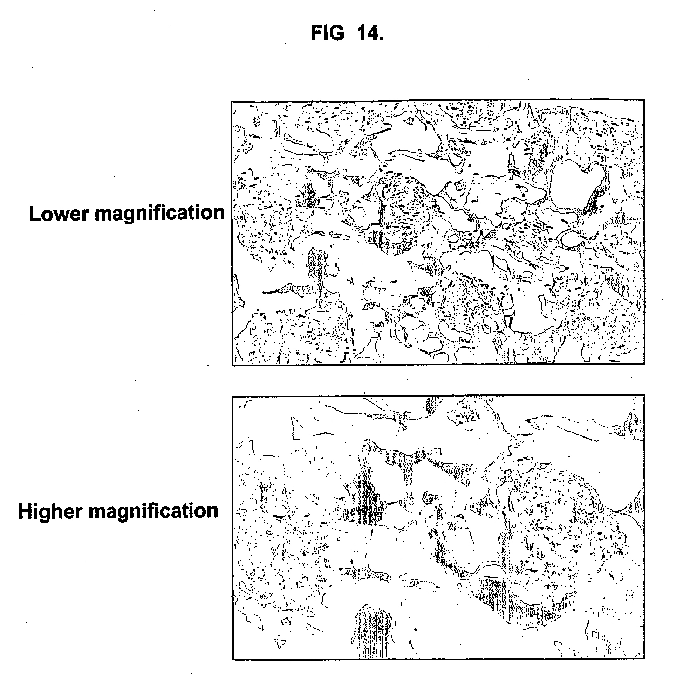

Dermal fibroblasts were seeded onto a gelatin sponge of diameter 3.5 cm, at a seeding density of about 2.5 x 10⁶ cells per cm² of sponge area, in DMEM + 10% FCS. This application of the high cell-seeding-density macromass culture method to the gelatin sponge resulted in formation of microscopic clusters of three-dimensional tissue-like organizations within the sponge, as seen in the histological section of the sponge, shown in FIG 14. Thus, the method of macromass culture can be used to generate three-dimensional tissue-like organization of cells at the microscopic level also, the tissue-like organizations becoming a component of the whole assembly.

### 9. CONCLUDING REMARKS:

The novelty of the present invention lies in the fact that high cell-seeding-density culture can generate whole three-dimensional tissue-like organization of cells without any specific agents that aid in tissue formation to induce such organization and can be scaled up areawise to generate macroscopic three-dimensional tissue-like constructs of different kinds, besides in the other important features such as the fact that scaffolding material is not employed or specific agents that aid in tissue formation/complex media formulations are not required, as are detailed in this description. The inventors of the present invention are the first to report that whole three-dimensional tissue-like organization of cells and macroscopic three-dimensional tissue-like constructs of different kinds can be generated by high cell-seeding-density culture alone.

It may be possible that other mesodermal cells or cells of endodermal or ectodermal origin can also form such tissue-like organization by macromass culture. Hence, tissue-like organization and macroscopic tissue-like constructs made by macromass culture from any type of mammalian cells, if they are possible, are within the scope of this invention, the defining feature being tissue-like organization achieved by the method of macromass culture.

While macroscopic tissue-like constructs are the preferred embodiment of the methods of this invention, it is apparent that macromass culture at a smaller scale, that is in culture areas anything smaller than about 0.75 cm diameter, would result in smaller tissue-like organizations, decreasing in size towards being microscopic as the scale of macromass culture is reduced. Microscopic tissue-like organization by high cell-seeding-density macromass culture in the above form or other form such as in the gelatin sponge described earlier is thus also within the scope of this invention. Likewise, tissue-like constructs could be generated by macromass culture in culture vessels larger than 8.5 cm diameter.

Thus, in the foregoing description, specific embodiments of this invention have been described examples have been presented with respect to the aspects of use of different cell types, the use of alternative growth surface, the use of change in medium to modulate the properties of the tissue formed, the scale-up of tissue formation, the sizes of culture vessels, the range of tissue-forming high cell densities, and the generation of a putative implant of which tissue made by macromass culture is a component. Although, only the described embodiments have been brought forth, they serve the purpose of example or illustration only, and do not limit the invention.

It should remain understood that different modifications or substitutions could be made to this invention, which would be within the scope of the present invention. For instance, it is contemplated by the inventors that one such modification would be the entrapment or encapsulation of tissue masses made by macromass culture into a suitable gel or matrix, or another such modification would be a construct in which multiple tissue-like masses or sheets are joined or held together by some means. In such constructs, the tissue-like construct made by macromass culture would now be a component of the whole substitute. The above are other ways, than those presented in the Results, by which tissue-like organization and constructs generated by macromass culture can be a component of an object. As demonstrated in the Results, even as it remains that tissue-like organization of cells by macromass culture does not require any scaffold and also histologically competent tissue-like constructs form without the aid of a scaffold, a scaffold may be provided for macromass culture, for example, as an alternative growth surface, such that the scaffold becomes a part of the whole substitute. Thus, the three-dimensional tissue-like organization and histologically competent tissue-like constructs can be developed scaffold-free, but can be combined with a suitable scaffold, if it gives beneficial properties or advantages over the tissue-like construct alone. Other modifications would be the use of other cell types which have the properties to form a tissue-like mass by macromass culture, use of other compatible growth surface than described, other medium changes for modulation, and other sizes of scale-up, etc. Therefore, this description of the present invention is not intended to limit this invention by the precise illustrative embodiments that are disclosed.

### REFERENCES CITED :

| | **U.S. PATENT DOCUMENTS** | |
|---|---|---|
| 5489304 | February 1996 | Orgill, *et al.* |
| 5460939 | October 1995 | Hansbrough, *et al.* |
| 5613982 | March 1997 | Goldstein. |
| 20020127711 | September 2002 | Kale, *et al.* |
| 5723331 | March 1998 | Tubo, *et al*. |

### OTHER REFERENCES

Abu-Absi SF, Friend JR, Hansen LK, Hu W (2002) Structural polarity and functional bile canaliculi in rat hepatocyte spheroids. Experimental Cell Research 274, 56-67.
Ahrens PB, Solursh M, Reiter RS (1977) Stage-related capacity for limb chondrogenesis in cell culture. Developmental Biology 60, 69-82.
Baldwin SP, Saltzman WM (2001) Aggregation enhances catecholamine secretion in cultured cells. Tissue Engineering 7(2)179-190.
Bancroft JD, Cook HC (1994) Manual of Histological Techniques and their Diagnostic Application, Churchill Livingstone, Edinburgh, UK.
Buttery LDK, Bourne S, Xynos JD, Wood H, Hughes FJ, Hughes SPF, Episkopou V, Polak JM (2001) Differentiation of osteoblasts and in vitro bone formation from murine embryonic stem cells. Tissue Engineering 7(1)89-99.
Davies CdeL, Berk DA, Pluen A, Jain RK (2002) Comparison of IgG diffusion and extracellular matrix composition in rhabdomyosarcomas grown in mice versus in vitro as spheroids reveals the role of host stromal cells. British Journal of Cancers 86, 1639-1644.
Denker AE, Nicoll SB, Tuan RS (1995) Formation of cartilage-like spheroids by micromass cultures of murine C3H10T1/2 cells upon treatment with transforming growth factor-beta 1. Differentiation 59(1)25-34.
Downie SA, Newman SA (1994) Morphogenetic differences between fore and hind limb precartilage mesenchyme : Relation to mechanisms of skeletal pattern formation. Developmental Biology 162, 195-208.
Eaglstein WH, Falanga V (1997) Tissue engineering and the development of Apligraf®, a human skin equivalent. Clinical Therapeutics 19(5)894-905.
Furukawa KS, Ushida T, Sakai Y, Suzuki M, Tanaka J, Tateishi T (2001) Formation of human fibroblast aggregates (spheroids) by rotational culture. Cell Transplantation 10, 441-445.
Hamamoto R, Yamada K, Kamihira M, Iijima S (1998) Differentiation and proliferation of primary rat hepatocytes cultured as spheroids. Journal of Biochemistry 124, 972-979.
Johnstone B, Hering TM, Caplan AI, Goldberg VM, Yoo JU (1998) In vitro chondrogenesis of bone marrow-derived mesenchymal progenitor cells. Experimental Cell Research 238, 265-272.
Kaji EH, Leiden JM (2001) Gene and stem cell therapies. Journal of the American Medical Association 285(5)545-550.
Kale S, Biermann S, Edwards C, Tamowski C, Morris M, Long MW (2000) Three-dimensional cellular development is essential for ex vivo formation of human bone. Nature Biotechnology 18, 954-958.
Kim HW, Han CD (2000) An overview of cartilage tissue engineering. Yonsei Medical Journal 41(6)766-773.
Lang SH, Stark M, Collins A, Paul AB, Stower MJ, Maitland NJ (2001) Experimental prostate epithelial morphogenesis in response to stroma and three-dimensional Matrigel culture. Cell Growth & Differentiation 12, 631-640.
L'Heureux N, Pâquet S, Labbé R, Germain L, Auger FA (1998) A completely biological tissue-engineered human blood vessel. FASEB Journal 12, 47-56.
Mackay AM, Beck SC, Murphy JM, Barry FP, Chichester CO, Pittenger MF (1998) Chondrogenic differentiation of cultured human mesenchymal stem cells from marrow. Tissue Engineering 4(4)415-428.
McPherson JM, Tubo R (2000) Articular cartilage injury. In Principles of Tissue Engineering, 2nd Edition, Academic Press, San Diego, USA, p.697-709.
Michel M, L'Heureux N, Pouliot R, Xu W, Auger FA, Germain L (1999) Characterization of a new tissue-engineered human skin equivalent with hair. In vitro Cellular & Developmental Biology-Animal 35, 318-326.
Pollok JM, Kluth D, Cusick RA, Lee H, Utsunomiya H, Ma PX, Langer R, Broelsch CE, Vacanti JP (1998) Formation of spheroidal aggregates of hepatocytes on biodegradable polymers under continuous flow bioreactor conditions. Journal of Pediatric Surgery 8, 195-199.
Service RF (2000) Tissue engineers build new bone. Science 289,1498-1500.
Shinji T, Koide N, Tsuji T (1988) Glycosaminoglycans partially substitute for proteoglycans in spheroid formation of adult rat hepatocytes in primary culture. Cell Structure & Function 13, 179-188.
Takezawa T, Mori Y, Yonaha T, Yoshizato K (1993) Characterization of morphology and cellular metabolism during the spheroid formation by fibroblasts. Experimental Cell Research 208,430-441.
Yamada K, Kamihira M, Hamamoto R, Iijima S (1998) Efficient induction of hepatocyte spheroids in a suspension culture using a water-soluble synthetic polymer as an artificial matrix. Journal of Biochemistry 123,1017-1023.
Yoon Y, Oh C, Kim D, Lee Y, Park J, Huh T, Kang S, Chun J (2000) Epidermal growth factor negatively regulates chondrogenesis of mesenchymal cells by modulating the protein kinase C-α, Erk-1, and p38 MAPK signaling pathways. Journal of Biological Chemistry 275(16)12353-12359.
Zuk PA, Zhu M, Mizuno H, Huang J, Futrell JW, Katz AJ, Benhaim P, Lorenz HP, Hedrick MH (2001) Multilineage cells from human adipose tissue : implications for cell-based therapies. Tissue Engineering 7(2)211-228.

## Claims

1. A method for the generation of a living tissue-like organisation of cells, including three-dimensional tissue-like constructs, without the aid of scaffold or extraneous matrix, the method comprising:
establishing a culture system in which cells that are not embryonic stem cells are seeded at a high density per unit area of a culture vessel at 3.33 x 10⁵ to 3 x 10⁶ cells per cm² resulting in a three-dimensional tissue-like formation or organisation of cells, without the aid of any agents that aid in tissue formation, such agents comprising tissue-inducing chemicals, tissue-inducing growth factors, substratum other than unmodified tissue culture plastic, or rotational culture; and
providing said tissue like constructs wherein optionally the cells are mesodermal cells.

2. The method according to claim 1, wherein the cells are selected from fibroblast cells, including dermal fibroblasts, osteoblast cells, adipose stromal cells, osteogenic cells derived from adipose stromal cells and chondrocytes.

3. The method according to claim 2, wherein fibroblast cells are derived from human skin tissue, chondrocytes are derived from human cartilage tissue, osteoblast cells are derived from human bone tissue, and adipose stromal cells are derived from human liposuction material.

4. The method according to claim 1, wherein said tissue-like organisation of cells is a tissue-like sheet comprising human fibroblastic cells of mesenchymal origin, the tissue-like sheet being free of synthetic or natural extraneous matrix that could cause an inflammatory reaction in some patients, and cellular so that it can produce growth factors and other proteins, and which can be prepared in a relatively short time.

5. The method according to claim 1, wherein the tissue-like organisation of cells includes macroscopic three-dimensional constructs suitable for use as tissue substitutes for implantation, for wound healing, or as *in vitro* models for drug testing, and the cells are fibroblastic cells of mesenchymal origin and optionally include:
a putative dermal equivalent made from dermal fibroblasts, putative substitute with bone-like properties made from osteogenic cells derived from adipose stromal cells, and putative cartilage substitute made from chondrocytes, wherein said cells are generated to assume different forms.

6. The method according to claim 5, wherein the tissue-like organisation of cells can be made to assume different forms, for the purpose of achieving different properties or qualities, said different forms comprising:
(a) three-dimensional macroscopic tissue-like constructs, wherein "macroscopic" means that the size of the tissue is at least such that it can be easily visually discerned by normal human vision, and the macroscopic tissue-like constructs are histologically competent; or
(b) the three-dimensional tissue-like organisation further combined with different matrices, such as gels, sheets, membranes or sponges or with other scaffolds

7. The method according to claim 6, wherein said matrices comprise collagen plus fibrin, gelatin sponge or nylon filter.

8. The method according to claim 5, wherein the tissue-like organisation of cells is generated wholly using cells alone and a culture medium.

9. The method according to claim 5, wherein the tissue-like organisation of cells is generated without the following agents that aid in tissue-formation: a bioreactor; extraneous scaffold or matrix or supports, or extraneous extracellular matrix components.

10. The method according to claim 5, wherein the tissue-like organisation of cells is generated from different cell types comprising:
dermal fibroblasts;
adipose stromal cells;
osteogenic cells derived from adipose stromal cells;
chondrocytes; and osteoblasts.

11. The method according to claim 5, wherein the exact tissue-forming high cell seeding density is different for different cell types.

12. The method according to claim 5, wherein the time taken to generate said different forms can vary for different cell types or different media conditions.

13. The method according to claim 5, wherein the tissue-like organisation of cells is generated without the aid of specific media conditions, including:
serum-free media conditions; and
complex media compositions.

14. The method according to claim 5, including the preparation of the tissue-like constructs in culture vessels of any shape, with a flat or curved base.

15. The method according to any one of claims 1 to 4, wherein all the seeded cells are integrated as a whole tissue-like organisation without any individual nodules.

16. The method according to claim 15, wherein the three-dimensional constructs are sheet-like, and/or larger than 7.5 mm in diameter.

17. The method according to claim 1, further comprising production of different tissue engineered products by generating tissue-like organisations of cells and formation of living, cellular putative tissue substitutes.

18. The method according to claim 17, wherein the tissue-like organisation is a macroscopic tissue-like construct.

19. The method according to claim 17, wherein high cell seeding density of 3.33 x 10⁵ to 3 x 10⁶ cells per cm² is achieved by settling the cells together within the three-dimensional space occupied by the cells at the base of the culture vessel such that they come into a state of close proximity with one another so as to trigger or signal them into a tissue formation mode by which they become cohesively integrated; and said range of cell seeding density is achieved in a vessel with a flat or curved base using a culture vessel of at least about 0.75 cm in diameter resulting in the formation of macroscopic tissue-like constructs, wherein macroscopic defines a tissue size that can be easily visually discerned by the normal human vision.

20. The method according to claim 1, further comprising the generation of a macroscopic, whole tissue-like organisation of cells suitable for implantation into a human or mammalian body, wherein the tissue-like organisation of cells is made from cells of mesenchymal origin such as (i) an engineered putative dermal equivalent made from dermal fibroblasts, (ii) putative substitute with bone-like properties made from adipose stromal cell-derived osteogenic cells or from osteoblasts or (iii) putative substitute for cartilage repair made from chondrocytes.

21. The method according to claim 20, wherein:
the tissue-like organisation of cells is suitable for implantation in a human or mammalian body as therapy for a disease or condition; and
the formation of the tissue-like organisation of cells does not require a preshaped well having a surface detrimental for cell attachment.

## Patentansprüche

1. Verfahren zur Erzeugung einer lebenden, gewebeartigen Organisation von Zellen, einschließlich dreidimensionaler gewebeartiger Konstrukte, ohne die Hilfe eines Gerüsts oder einer Fremdmatrix, wobei das Verfahren folgendes umfasst:
das Herstellen eines Kultursystems, in dem Zellen, die nicht embryonale Stammzellen sind, in einer hohen Dichte pro Flächeneinheit eines Kulturgefäßes bei 3,33 x 10⁵ bis 3 x 10⁶ Zellen pro cm² ausgesät werden, wobei eine dreidimensionale, gewebeartige Formation oder Organisation von Zellen entsteht, ohne die Hilfe von jeglichen Mitteln, die bei der Gewebebildung helfen, wobei solche Mittel Gewebe-induzierende Chemikalien, Gewebe-induzierende Wachstumsfaktoren, andere Substrate als unmodifizierter Gewebekulturkunststoff oder Rotationskultur umfassen, und
das Zurverfügungstellen der gewebeartigen Konstrukte, wobei optional die Zellen Mesodermzellen sind.

2. Verfahren nach Anspruch 1, wobei die Zellen aus Fibroblastzellen, einschließlich dermalen Fibroblasten, Osteoblastzellen, fetthaltigen Stromazellen, von fetthaltigen Stromazellen abstammende osteogenen Zellen und Chondrozyten ausgewählt werden.

3. Verfahren nach Anspruch 2, wobei die Fibroblastzellen von menschlichem Hautgewebe abstammen, die Chondrozyten von menschlichem Knorpelgewebe abstammen, die Osteoblastzellen von menschlichem Knochengewebe abstammen und die fetthaltigen Stromazellen von menschlichem Material aus abgesaugten Fett abstammen.

4. Verfahren nach Anspruch 1, wobei die gewebeartige Organisation von Zellen eine gewebeartige dünne Schicht ist, die menschliche fibroblastische Zellen mesenchymalen Ursprungs umfasst, wobei die gewebeartige dünne Schicht frei von synthetischer oder natürlicher Fremdmatrix ist, die eine entzündliche Reaktion in manchen Patienten hervorrufen könnte, und zellulär ist, so dass sie Wachstumsfaktoren und andere Proteine produzieren kann, und welche in einer relativ kurzen Zeit hergestellt werden kann.

5. Verfahren nach Anspruch 1, wobei die gewebeartige Organisation von Zellen makroskopische dreidimensionale Konstrukte beinhaltet, die zur Verwendung als Gewebeersatz für die Implantation, zur Wundheilung oder als in vitro Modelle für das Testen von Arzneistoffen geeignet sind, und die Zellen fibroblastische Zellen mesenchymalen Ursprungs sind und optional folgendes beinhalten:
ein mutmaßliches dermales Äquivalent, gemacht aus dermalen Fibroblasten, ein mutmaßlicher Ersatz mit knochenartigen Eigenschaften, gemacht aus von fetthaltigen Stromazellen abstammende osteogenen Zellen, und ein mutmaßlicher Knorpelersatz, gemacht aus Chondrozyten, wobei die Zellen erzeugt werden, um verschiedene Formen anzunehmen.

6. Verfahren nach Anspruch 5, wobei die gewebeartige Organisation von Zellen gemacht werden kann, um verschiedene Formen anzunehmen, für den Zweck des Erreichens verschiedener Eigenschaften oder Qualitäten, wobei die verschiedenen Formen folgendes umfassen:
(a) dreidimensionale makroskopische gewebeartige Konstrukte, wobei "makroskopisch" bedeutet, dass die Größe des Gewebes zumindest dergestalt ist, dass es durch normale menschliche Sehkraft leicht visuell wahrnehmbar ist, und die makroskopischen gewebeartigen Konstrukte histologisch funktionsfähig-sind, oder
(b) die dreidimensionale gewebeartige Organisation, die ferner mit verschiedenen Matrices, wie zum Beispiel Gelen, dünne Schichten, Membranen oder Schwämmen oder mit anderen Gerüsten kombiniert ist.

7. Verfahren nach Anspruch 6, wobei die Matrices Collagen plus Fibrin, Gelatineschwamm oder Nylonfilter umfassen.

8. Verfahren nach Anspruch 5, wobei die gewebeartige Organisation von Zellen gänzlich unter Verwendung von Zellen alleine und einem Kulturmedium erzeugt wird.

9. Verfahren nach Anspruch 5, wobei die gewebeartige Organisation von Zellen ohne die folgenden Mittel, die bei der Gewebebildung helfen, erzeugt wird: einem Bioreaktor, Fremdgerüst oder -matrix oder -träger, oder fremdartige extrazelluläre Matrixkomponenten.

10. Verfahren nach Anspruch 5, wobei die gewebeartige Organisation von Zellen aus verschiedenen Zelltypen erzeugt wird, die dermale Fibroblasten, fetthaltige Stromazellen, von fetthaltigen Stromazellen abstammende osteogene Zellen, Chondrozyten und Osteoblasten umfassen.

11. Verfahren nach Anspruch 5, wobei die genaues gewebebildende, hohe Zellsaatdichte für verschiedene Zelltypen verschieden ist.

12. Verfahren nach Anspruch 5, wobei die Zeit, die für die Erzeugung der verschiedenen Formen aufgebracht wird, für die verschiedenen Zelltypen oder verschiedenen Bedingungen der Medien variieren kann.

13. Verfahren nach Anspruch 5, wobei die gewebeartige Organisation von Zellen ohne die Hilfe von spezifischen Bedingungen der Medien erzeugt wird, einschließlich Bedingungen von serumfreien Medien und Bedingungen von komplexen Medien.

14. Verfahren nach Anspruch 5, das die Herstellung von gewebeartigen Konstrukten in Kulturgefäßen jeglicher Gestalt, mit einem flachen oder einem gebogenen Boden, beinhaltet.

15. Verfahren nach einem der Ansprüche 1 bis 4, wobei alle gesäte Zellen als eine gemeinsame gewebeartige Organisation ohne jegliche individuelle Knötchen zusammengeschlossen sind.

16. Verfahren nach Anspruch 15, wobei die dreidimensionalen Konstrukte blattartig sind und/oder größer als 7,5 mm im Durchmesser sind.

17. Verfahren nach Anspruch 1, das ferner die Herstellung von verschiedenen gewebekonstruierten Produkten durch Erzeugung gewebeartiger Organisationen von Zellen und die Bildung von lebendem, zellulären mutmaßlichen Gewebeersatz umfasst.

18. Verfahren nach Anspruch 17, wobei die gewebeartige Organisation ein makroskopisches gewebeartiges Konstrukt ist.

19. Verfahren nach Anspruch 17, wobei die hohe Zellsaatdichte von 3,33 x 10⁵ bis 3 x 10⁶ Zellen pro cm² erreicht wird durch ein gemeinsames Absetzen der Zellen innerhalb des dreidimensionalen Raumes-, der durch die Zellen am Boden des Kulturgefäßes belegt ist, sodass sie in einen Zustand von enger Nähe miteinander kommen, sodass ein Gewebsbildungsmodus getriggert oder ihnen signalisiert wird, durch den sie kohäsiv zusammengeschlossen werden; und wobei der Bereich der Zellsaatdichte erreicht wird in einem Gefäß mit flachem oder gebogenem Boden unter Verwendung eines Kulturgefäßes von mindestens ungefähr 0,75 cm im Durchmesser, woraus die Bildung von makroskopischen gewebeartigen Konstrukten resultiert, wobei makroskopisch eine Gewebsgröße bedeutet, die durch die normale menschliche Sehkraft einfach visuell wahrgenommen werden kann.

20. Verfahren nach Anspruch 1, das ferner die Erzeugung einer makroskopischen, ganzen gewebeartigen Organisation von Zellen umfasst, die zur Implantation in einen menschlichen Körper oder einen Säugerkörper geeignet ist, wobei die gewebeartige Organisation von Zellen aus Zellen mesenchymalen Ursprungs gemacht ist, wie zum Beispiel (i) einem konstruierten mutmaßlichen dermalen Äquivalent, gemacht aus dermalen Fibroblasten, (ii) einem mutmaßlichen Ersatz mit knochenähnlichen Eigenschaften, gemacht aus von fetthaltigen Stromazellen abstammende osteogenen Zellen oder von Osteoblasten oder (iii) einem mutmaßlichen Ersatz für die Reparatur von Knorpel, gemacht aus Chondrocyten.

21. Verfahren nach Anspruch 20, wobei
die gewebeartige Organisation von Zellen für die Implantation in einen menschlichen Körper oder einen Säugerkörper als Therapie für eine Krankheit oder einen Zustand geeignet ist, und
die Bildung der gewebeartigen Organsiation von Zellen nicht eine vorgeformte Vertiefung bedarf, die eine Oberfläche hat, die für das Anhaften von Zellen schädlich ist.

## Revendications

1. Procédé pour produire une organisation de cellules analogue à un tissu vivant, incluant des constructions analogues à un tissu tridimensionnelles, sans l'aide d'échafaudage ou de matrice extracellulaire, le procédé comprenant :
l'établissement d'un système de culture dans lequel des cellules qui ne sont pas des cellules souches embryonnaires sont semées à une haute densité par unité d'aire d'un récipient de culture à raison de 3,33 x 10⁵ à 3 x 10⁶ cellules par cm² conduisant à une formation ou organisation de cellules analogue à un tissu tridimensionnelle, sans l'aide de quelconques agents favorisant la formation d'un tissu, de tels agents comprenant des produits chimiques induisant un tissu, des facteurs de croissance induisant un tissu, un substrat différent d'une matière plastique de culture de tissu non modifiée, ou la culture en rotation ; et
la fourniture desdites constructions analogues à un tissu où éventuellement les cellules sont des cellules mésodermiques.

2. Procédé selon la revendication 1 où les cellules sont choisies parmi les cellules fibroblastes, incluant les fibroblastes thermiques, les cellules ostéoblastes, les cellules stromales adipeuses, les cellules ostéogènes dérivées de cellules stromales adipeuses et les chondrocytes.

3. Procédé selon la revendication 2 où les cellules fibroblastes sont dérivées de tissu cutané humain, les chondrocytes sont dérivées de tissu cartilagineux humain, les cellules ostéoblastes sont dérivées de tissu osseux humain, et les cellules stromales adipeuses sont dérivées de matériel de liposuccion humain.

4. Procédé selon la revendication 1 où ladite organisation de cellules analogue à un tissu est une feuille analogue à un tissu comprenant des cellules fibroblastiques humaines d'origine mésenchymateuse, la feuille analogue à un tissu étant dépourvue de matrice étrangère synthétique ou naturelle qui pourrait provoquer une réaction inflammatoire chez certains patients, et cellulaire de sorte qu'elle peut produire des facteurs de croissance et d'autres protéines, et qui peut être préparée en une durée relativement courte.

5. Procédé selon la revendication 1 où l'organisation de cellules analogue à un tissu inclut des constructions tridimensionnelles macroscopiques appropriées pour être utilisées comme succédanés de tissu pour l'implantation, pour la cicatrisation des plaies, ou comme modèles *in vitro* pour tester des médicaments, et les cellules sont des cellules fibroblastiques d'origine mésenchymateuse et incluent éventuellement :
un équivalent dermique supposé produit à partir de fibroblastes dermiques, un succédané supposé ayant des propriétés analogues aux os produit à partir de cellules ostéogènes dérivées de cellules stromales adipeuses et un succédané de cartilage supposé produit à partir de chondrocytes, où lesdites cellules sont produites pour revêtir différentes formes.

6. Procédé selon la revendication 5 où l'organisation de cellules analogue à un tissu peut être amenée à revêtir différentes formes dans le but d'obtenir différentes propriétés ou qualités, lesdites différentes formes comprenant :
(a) les constructions analogues à un tissu macroscopiques tridimensionnelles, où "macroscopique" signifie que la taille du tissu est au moins telle qu'il peut être discerné visuellement aisément par la vision humaine normale, et les constructions analogues à un tissu macroscopiques sont histologiquement compétentes ; ou
(b) l'organisation analogue à un tissu tridimensionnelle combinée en outre avec différentes matrices, comme des gels, des feuilles, des membranes ou des éponges ou avec d'autres échafaudages.

7. Procédé selon la revendication 6 où lesdites matrices comprennent du collagène plus de la fibrine, une éponge de gélatine ou un filtre en Nylon,

8. Procédé selon la revendication 5 où l'organisation de cellules analogue à un tissu est produite totalement au moyen de cellules seules et d'un milieu de culture.

9. Procédé selon la revendication 5 où l'organisation de cellules analogue à un tissu est produite sans les agents suivants qui favorisent la formation d'un tissu : un bioréacteur ; un échafaudage ou une matrice ou des supports étrangers, ou des composants de matrice extracellulaire étrangers.

10. Procédé selon la revendication 5 où l'organisation de cellules analogue à un tissu est produite à partir de différents types de cellules comprenant :
des fibroblastes dermiques ;
des cellules stromales adipeuses ;
des cellules ostéogènes dérivées de cellules stromales adipeuses ;
des chondrocytes ; et des ostéoblastes.

11. Procédé selon la revendication 5 où la haute densité d'ensemencement des cellules formant un tissu exacte est différente pour des types de cellules différents.

12. Procédé selon la revendication 5 où la durée nécessaire pour produire lesdites différentes formes peut varier pour des types de cellules différents ou des conditions de milieu différentes.

13. Procédé selon la revendication 5 où l'organisation de cellules analogue à un tissu est produite sans l'aide de conditions de milieu spécifiques incluant :
des conditions de milieu sans sérum ; et
des compositions de milieux complexes.

14. Procédé selon la revendication 5 incluant la préparation des constructions analogues à un tissu dans des récipients de culture de forme quelconque, avec une base plane ou incurvée.

15. Procédé selon l'une quelconque des revendications 1 à 4 où toutes les cellules semées sont intégrées sous forme d'une organisation analogue à un tissu complète sans aucun nodule individuel.

16. Procédé selon la revendication 15 où les constructions tridimensionnelles sont analogues à des feuilles, et/ou ont un diamètre supérieur à 7,5 mm.

17. Procédé selon la revendication 1 comprenant en outre la production de différents produits modifiés de manière tissulaire par production d'organisations analogue à un tissu de cellules et formation de succédanés tissulaires supposés cellulaires vivants.

18. Procédé selon la revendication 17 où l'organisation analogue à un tissu est une construction analogue à un tissu macroscopique.

19. Procédé selon la revendication 17 où une haute densité d'ensemencement de cellules de 3,33 x 10⁵ à 3 x 10⁶ cellules par cm² est obtenue par dépôt des cellules ensemble dans l'espace tridimensionnel occupé par les cellules à la base du récipient de culture de sorte qu'elles passent dans un état de proximité étroite entre elles de manière à déclencher ou signaler en elles un mode de formation de tissu par lequel elles deviennent intégrées de manière cohésive ; et ladite plage de densité d'ensemencement des cellules est obtenue dans un récipient ayant une base plane ou incurvée au moyen d'un récipient de culture d'un diamètre d'au moins environ 0,75 cm conduisant à la formation de constructions analogues à un tissu macroscopiques, où macroscopique définit une taille de tissu qui peut être aisément discernée visuellement par la vision humaine normale.

20. Procédé selon la revendication 1 comprenant en outre la production d'une organisation de cellules analogue à un tissu complète macroscopique appropriée pour l'implantation dans un organisme humain ou de mammifère, où l'organisation de cellules analogue à un tissu est produite à partir de cellules d'origine mésenchymateuse comme (i) un équivalent dermique supposé modifié produit à partir de fibroblastes dermiques ; (ii) un succédané supposé ayant des propriétés analogues aux os produit à partir de cellules ostéogènes dérivées de cellules stromales adipeuses ou à partir d'ostéoblastes ou (iii) un succédané supposé pour la réparation du cartilage produit à partir de chondrocytes.

21. Procédé selon la revendication 20, où :
l'organisation de cellules analogue à un tissu est appropriée pour l'implantation dans un organisme humain ou de mammifère comme thérapie pour une maladie ou affection ; et
la formation de l'organisation de cellule analogue à un tissu n'exige pas un puits préformé ayant une surface défavorable à la fixation des cellules.
